(19) Europäisches Patentamt
European Patent Office
Office européen des brevets

(11) Publication number: 0 269 452 A2

(12) EUROPEAN PATENT APPLICATION

(21) Application number: 87310472.3

(22) Date of filing: 27.11.87

(51) Int. Cl.[4]: A 61 K 31/445
//(A61K31/445,31:415)

(30) Priority: 28.11.86 GB 8628475

(43) Date of publication of application: 01.06.88 Bulletin 88/22

(84) Designated Contracting States: AT BE CH DE ES FR GB GR IT LI LU NL SE

(71) Applicant: GLAXO GROUP LIMITED
Clarges House 6-12 Clarges Street
London W1Y 8DH (GB)

(72) Inventor: Tyers, Michael Brian
c/o Glaxo Group Research Limited Priory Street
Ware Hertfordshire (GB)

(74) Representative: Marchant, James Ian et al
Elkington and Fife High Holborn House 52/54 High Holborn
London WC1V 6SH (GB)

(54) **Use of ketone derivatives for the manufacture of medicaments.**

(57) A medicament for use in the treatment of gastrointestinal disorders comprising 1,2,3,9-tetrahydro-9-methyl-3-[(2-methyl-1H-imidazol-1-yl)methyl]-4H-carbazol-4-one or a physiologically acceptable salt or solvate thereof and sufotidine or a physiologically acceptable salt thereof.

EP 0 269 452 A2

**Description**

USE OF KETONE DERIVATIVES FOR THE MANUFACTURE OF MEDICAMENTS

This invention relates to improvements in the treatment of gastrointestinal disorders. More particularly it relates to the use of a compound having antagonist activity at $5HT_3$ receptors and sufotidine in the treatment of such conditions, and to pharmaceutical compositions containing the two compounds.

In our UK Patent Specification No. 2153821A we disclose inter alia 1,2,3,9-tetrahydro-9-methyl-3-[(2-methyl-1H-imidazol-1-yl)methyl]-4H-carbazol-4-one which may be represented by the formula (I)

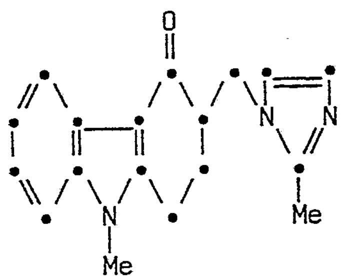

(I)

and physiologically acceptable salts, solvates and physiologically acceptable equivalents thereof.

In the aforementioned specification the compounds are described as potent and selective antagonists of 5-hydroxytryptamine (5HT) at 'neuronal' 5HT receptors of the type located on terminals of primary afferent nerves, and which are also present in the central nervous system. Receptors of this type are now designated $5HT_3$ receptors. The compounds are described as being of use in the treatment of a human or animal subject suffering from a condition caused by a disturbance of neuronal 5HT function, for example in the treatment of migraine pain or a psychotic disorder such as schizophrenia. The compounds may also be useful in the treatment of conditions such as anxiety, obesity and mania.

We have found a compound of formula (I) additionally promotes gastric emptying and is thus useful in the treatment of conditions which may be relieved by the promotion of gastric emptying. Such conditions include gastric stasis and symptoms of gastrointestinal dysfunction such as dyspepsia, reflux oesophagitis, peptic ulcer and flatulence.

The compound of formula (I) has also been found to be an anti-emetic, and may be used in the treatment or prevention of nausea and vomiting. The use of the compound of formula (I) for the treatment of emesis is described in published European Patent Specification No. 201165, which specification also refers to the use of the compound of formula (I) for the treatment of irritable bowel syndrome.

Sufotidine is the approved name for 1-methyl-3-methylsulphonylmethyl-N-[3-[3-(1-piperidinylmethyl)phenoxy]propyl]-1H-1,2,4-triazole-5-amine which is described and claimed in British Patent Specification No. 2075007B. It is a potent histamine $H_2$-antagonist which may be used in the treatment of conditions where there is an advantage in lowering gastric acidity. Such conditions include duodenal and gastric ulceration, reflux oesophagitis and Zollinger-Ellison syndrome. Sufotidine may also be used prophylactically in surgical procedures, and in the treatment of allergic and inflammatory conditions were histamine is a known mediator.

The combination of the $5HT_3$ receptor antagonist of formula (I), with the $H_2$-receptor antagonist sufotidine, provides a useful and advantageous combination for the treatment of gastrointestinal disorders. Administration of the compound of formula (I) and sufotidine is particularly useful for the treatment of conditions such as reflux oesophagitis where the promotion of gastric emptying serves to alleviate the reflux, thereby encouraging the healing effect of the $H_2$-receptor antagonist. Co-administration of the compound of formula (I) and sufotidine may also be useful in general anaesthesia. More particularly, when the two compounds are given before or during anaesthesia, the promotion of gastric emptying by the $5HT_3$ antagonist and the reduction of gastric acid production by sufotidine prevent both acid inhalation during anaesthesia and post-anaesthetic nausea and vomiting. Co-administration of the compound of formula (I) and sufotidine may also be useful for the treatment of irritable bowel syndrome.

The present invention thus provides a method of treating a condition which may be relieved by the promotion of gastric emptying and/or relieving nausea and vomiting, which comprises administering to a human or animal subject the compound of formula (I) or a physiologically acceptable salt or solvate thereof, and sufotidine or a physiologically acceptable salt thereof.

According to another aspect the invention provides for the use of the $5HT_3$ receptor antagonist of formula (I) or a physiologically acceptable salt or solvate thereof for the manufacture of a medicament for administration in conjunction with sufotidine or a physiologically acceptable salt thereof, for the treatment of a condition which may be relieved by the promotion of gastric emptying and/or the relief of nausea and vomiting.

The compound of formula (I) or a physiologically acceptable salt or solvate thereof, and sufotidine or a physiologically acceptable salt thereof, may be administered as a single pharmaceutical composition comprising effective amounts of the two active ingredients. Alternatively the two active ingredients may be co-administered in the form of two separate pharmaceutical compositions for simultaneous or sequential use.

Suitable physiologically acceptable salts of the carbazolone of formula (I) for use according to the invention include acid addition salts formed with organic or inorganic acids for example, hydrochlorides, hydrobromides, sulphates, phosphates, citrates, fumarates and maleates. The solvates may, for example, be hydrates. A preferred form of the compound of formula (I) for use according to the invention is the hydrochloride, particularly in hydrated form, e.g. the dihydrate.

The use of physiologically acceptable equivalents of the compound of formula (I), i.e. a physiologically acceptable compound which is converted in vivo into the parent compound of formula (I), is also included within the scope of the invention.

Sufotidine may be administered according to the invention in the form of either its free base or a physiologically acceptable salt. Suitable salts include salts of inorganic or organic acids such as the hydrochloride, hydrobromide, sulphate, methanesulphonate, acetate, maleate, succinate, citrate, tartrate, benzoate and fumarate salts. Sufotidine as its free base represents a preferred form for administration.

A proposed dosage of the compound of formula (I) for use according to the invention for administration to man (of approximately 70kg body weight), is 0.05 to 25mg, more preferably 0.05 to 20mg, and most preferably 0.1 to 10mg per unit dose, expressed as the weight of free base.

A preferred dose of sufotidine for use according to the invention is in the range of 50-600mg per dosage unit, expressed as the weight of free base.

The unit doses may be administered, for example, 1 to 4 times per day. The exact dose will depend on the route of administration and the condition being treated, and it will be appreciated that it may be necessary to make routine variations to the dosage depending on the age and weight of the patient as well as the severity of the condition to be treated.

According to a further aspect the invention provides a pharmaceutical composition, for use in human or veterinary medicine, comprising the compound of formula (I) or a physiologically acceptable salt or solvate thereof, and sufotidine or a physiologically acceptable salt thereof.

Compositions containing the compound of formula (I) in the form of a physiologically acceptable equivalent, as defined above, are also included within this aspect of the invention.

Compositions according to the invention may be formulated in conventional manner using one or more physiologically acceptable carriers or excipients. Thus the compositions may, for example, be formulated for oral, buccal, parenteral or rectal administration.

Particularly useful pharmaceutical compositions for use according to the invention are those in a form suitable for oral or rectal administration.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetted agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of one or both active ingredients.

For buccal administration the compositions may take the form of tablets or lozenges formulated in conventional manner.

For parenteral administration the compositions may be presented in a form suitable for bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredients may be in powder form for constitution with a suitable vehicle, e.g. sterile pyrogen-free water, before use.

For rectal administration the compositions may be formulated as suppositories or retention enemas, e.g. containing conventional suppository bases such as cocoa butter or other glycerides.

In addition to the formulations described previously, the compositions according to the invention may also take the form of depot preparations. Such long acting formulations may be administered by implantation (for example subcutaneously or intramuscularly) or by intramuscular injection. Thus, for example, the active ingredients may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions of the invention may be prepared according to conventional techniques well known in the pharmaceutical industry. Thus, for example, the compound of formula (I) or a salt or solvate thereof and the sufotidine or sufotidine salt may be admixed together, if desired, with suitable excipients.

Tablets may be prepared, for example, by direct compression of such a mixture. Capsules may be prepared by filling the blend along with suitable excipients into gelatin capsules, using a suitable filling machine. Controlled release forms for oral or rectal administration may be formulated in a conventional manner associated with controlled release forms.

The compositions for use according to the invention may, if desired, be presented in a pack or dispenser which may contain one or more unit dosage forms containing the active ingredients. The pack may for example comprise metal or plastic foil, such as a blister pack. The pack or dispenser device may be accompanied by instructions for administration. Where the compound of formula (I) and the sufotidine are intended for administration as two separate compositions these may be presented in the form of, for example, a twin pack.

The following examples illustrate the preparation of the compound of formula (I). Temperatures are in °C.

Example 1

1,2,3,9-Tetrahydro-9-methyl-3-[(2-methyl-1H-imidazol-1-yl)methyl]-4H-carbazol-4-one

A solution of 3-[(dimethylamino)methyl]-1,2,3,9-tetrahydro-9-methyl-4H-carbazol-4-one hydrochloride (1.7g) in water (17ml) was treated with 2-methylimidazole (1.4g) and then heated under reflux for 20h. The cooled mixture was filtered and the residue washed with water (3x15ml) to give a product (1.7g) m.p. 221-221.5°. This material was recrystallised from methanol to give the title compound (1.4g) m.p. 231-232°.

Example 2

1,2,3,9-Tetrahydro-9-methyl-3-[(2-methyl-1H-imidazol-1-yl)methyl]-4H-carbazol-4-one hydrochloride dihydrate

1,2,3,9-Tetrahydro-9-methyl-3-[(2-methyl-1H-imidazol-1-yl)methyl]-4H-carbazol-4-one (18.3g) in a hot mixture of isopropanol (90ml) and water (18.3ml) was treated with concentrated hydrochloric acid (6.25ml). The hot mixture was filtered and the filtrate diluted with isopropanol (90ml) and stirred at room temperature for 17h, cooled to 2° and the solid filtered off (21.6g). A sample (6g) was recrystallised from a mixture of water (6ml) and isopropanol (10ml) to give the title compound as a white crystalline solid (6g) m.p. 178.5-179.5°.

Analysis Found: C,59.45;H,6.45;N,11.5.
$C_{18}H_{19}N_3O.HCl.2H_2O$ requires C,59.1;H,6.6;N,11.5%.
Water assay Found: 10.23%
$C_{18}H_{19}N_3O.HCl.2H_2O$ requires 9.85%

The following examples illustrate pharmaceutical compositions according to the invention, containing 1,2,3,9-tetrahydro-9-methyl-3-[(2-methyl-1H-imidazol-1-yl)methyl]-4H-carbazol-4-one hydrochloride dihydrate (Compound A) and sufotidine (free base) as the active ingredients. Other physiologically acceptable salts and/or solvates of the compound of formula (I), and sufotidine or physiologically acceptable salts thereof, may be formulated in a similar manner.

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques.

| Direct Compression Tablet | mg/tablet |
|---|---|
| Compound A | 5.00* |
| Sufotidine | 100.00 |
| Microcrystalline cellulose NF | 100.00 |
| Anhydrous lactose NF | 93.50 |
| Magnesium stearate BP | 1.50 |
| Compression weight | 300.00 |

* Equivalent to 4.0mg free base.

Compound A and the sufotidine are blended with the excipients. The resultant mix is compressed into tablets using a suitable tablet press fitted with 9.5mm normal concave punches.

| Wet Granulation Tablet | mg/tablet |
|---|---|
| Compound A | 10.0 * |
| Sufotidine | 200.0 |
| Lactose BP | 126.0 |
| Starch BP | 40.0 |
| Pregelatinised Maize Starch BP | 20.0 |
| Magnesium Stearate BP | 4.0 |
| Compression Weight | 400.0 |

* Equivalent to 8.0mg free base.

The active ingredients are sieved through a suitable sieve and blended with lactose, starch and pregelatinised maize starch. Suitable volumes of purified water are added and the powders are granulated. After drying, the granules are screened and blended with the magnesium stearate. The granules are then compressed into tablets using 11mm diameter punches.

Tablets of other strengths and/or combination of doses may be prepared by appropriate alterations in the amounts of the active ingredients and the excipients and using punches to suit.

CAPSULES

| | mg/capsule |
|---|---|
| Compound A | 10.0 |
| Sufotidine | 200.0 |
| Microcrystalline Cellulose NF | 87.0 |
| Magnesium Stearate BP | 3.0 |
| Fill Weight | 300.0 |

The active ingredients are sieved and blended with the excipients. The mix is filled into size No. 0 hard gelatin capsules using suitable machinery. Other doses may be prepared by altering the fill weight and if necessary changing the capsule size to suit.

SYRUP

This may be either a sucrose or sucrose free presentation.

| A. Sucrose Syrup | | mg/5mℓ dose |
|---|---|---|
| Compound A | | 5.0 |
| Sufotidine | | 100.0 |
| Sucrose BP | | 2750.0 |
| Glycerine BP | | 500.0 |
| Buffer ) | | |
| Flavour ) | | as required |
| Colour ) | | |
| Preservative ) | | |
| Purified Water BP | to | 5.0mℓ |

The active ingredients, buffer, flavour, colour and preservative are dissolved in some of the water and the glycerine is added. The remainder of the water is heated to dissolve the sucrose and is then cooled. The two solutions are combined, adjusted to volume and mixed. The syrup is clarified by filtration.

| B. Sucrose-Free | | mg/5mℓ dose |
|---|---|---|
| Compound A | | 5.0 |
| Sufotidine | | 100.0 |
| Hydroxypropylmethylcellulose USP (viscosity type 4000) | | 22.5 |
| Buffer ) | | |
| Flavour ) | | |
| Colour ) | | As required |
| Preservative ) | | |
| Sweetener ) | | |
| Purified Water BP | to | 5.0mℓ |

The hydroxypropylmethylcellulose is dispersed in hot water, cooled and then mixed with an aqueous solution containing the active ingredients and the other components of the formulation. The resultant solution is adjusted to volume and mixed. The syrup is clarified by filtration.

SUPPOSITORY

| | |
|---|---|
| Compound A | 10.0mg |
| Sufotidine | 200.0mg |
| * Witepsol H15 to | 1.5g |

* A proprietory grade of Adeps Solidus Ph. Eur.

A suspension of the active ingredients in molten Witepsol is prepared and filled into appropriate suppository moulds using suitable machinery.

## Claims

1. A pharmaceutical composition for use in human or veterinary medicine comprising 1,2,3,9-tetrahydro-9-methyl-3-[(2-methyl-1H-imidazol-1-yl)methyl]-4H-carbazol-4-one or a physiologically acceptable salt or solvate thereof and sufotidine or a physiologically acceptable salt thereof.

2. A pharmaceutical composition as claimed in claim 1 wherein the 1,2,3,9-tetrahydro-9-methyl-3-[(2-methyl-1H-imidazol-1-yl)methyl]-4H-carbazol-4-one is used in the form of the hydrochloride dihydrate.

3. A pharmaceutical composition as claimed in claim 1 or 2 wherein the sufotidine is used in the form of the free base.

4. A pharmaceutical composition as claimed in any of claims 1 to 3 in unit dose form containing 0.05 to 25 mg per unit dose of 1,2,3,9-tetrahydro-9-methyl-3-[(2-methyl-1H-imidazol-1-yl)methyl]-4H-carbazol-4-one and 50 to 600 mg per unit dose of sufotidine, both doses being expressed as the weight of free base.

5. A pharmaceutical composition as claimed in claim 4 containing 0.05 to 20 mg per unit dose of 1,2,3,9-tetrahydro-9-methyl-3-[(2-methyl-1H-imidazol-1-yl)methyl]-4H-carbazol-4-one.

6. A pharmaceutical composition as claimed in any of claims 1 to 5 in a form adapted for oral, buccal, parenteral or rectal administration.

7. A pharmaceutical composition as claimed in claim 6 for oral administration in the form of tablets.

8. Use of 1,2,3,9-Tetrahydro-9-methyl-3-[(2-methyl-1H-imidazol-1-yl)methyl]-4H-carbazol-4-one and physiologically acceptable salts and solvates thereof for the manufacture of a medicament for administration in conjunction with sufotidine or a physiologically acceptable salt thereof in the treatment of a condition which may be relieved by the promotion of gastric emptying and/or the relief of nausea and vomiting.

9. Use according to claim 8 in the manufacture of a medicament for administration in conjunction with sufotidine or a physiologically acceptable salt thereof in the treatment of a condition which may be relieved by the promotion of gastric emptying.

10. Use according to claim 8 or 9 wherein the 1,2,3,9-tetrahydro-9-methyl-3-[(2-methyl-1H-imidazol-1-yl)methyl]-4H-carbazol-4-one is used in a unit dose of 0.05 to 20 mg and the sufotidine in a unit dose of 50 to 600 mg, both doses being expressed as the weight of free base.

11. Use according to any of claims 8 to 10 wherein the 1,2,3,9-tetrahydro-9-methyl-3-[(2-methyl-1H-imidazol-1-yl)methyl]-4H-carbazol-4-one is used in the form of the hydrochloride dihydrate.

12. Use according to any of claims 8 to 11 wherein the sufotidine is used in the form of the free base.

13. A twin pack comprising separate unit dosage forms of 1,2,3,9-tetrahydro-9-methyl-3-[(2-methyl-1H-imidazol-1-yl)methyl]-4H-carbazol-4-one or a physiologically acceptable salt or solvate thereof and sufotidine or a physiologically acceptable salt thereof in association for separate administration.